# EUROPÄISCHE PATENTSCHRIFT

(11) **EP 3 556 211 B1**
(45) Veröffentlichungstag und Bekanntmachung des Hinweises auf die Patenterteilung: **25.01.2023**
(21) Anmeldenummer: 19157188.4
(22) Anmeldetag: 12.08.2015
(51) Int. Cl.: A01N 25/02, A01P 13/00, A01N 47/36, A01N 41/10, A01N 37/02, A01N 39/04, A01N 37/40, A01N 33/12, A01N 25/22

(54) **ORGANISCHE AMMONIUMSALZE VON ANIONISCHEN PESTIZIDEN**
ORGANIC AMMONIUM SALTS OF ANIONIC PESTICIDES
SELS D'AMMONIUM ORGANIQUES DE PESTICIDES ANIONIQUES

(30) Priorität: 13.08.2014 DE 102014012022
(43) Veröffentlichungstag der Anmeldung: 23.10.2019
(62) Teilanmeldung aus: 15753923.0
(73) Patentinhaber: Clariant International Ltd, 4132 Muttenz (CH)
(72) Erfinder: BAUR, Peter, 86938 Schondorf (DE); KLUG, Peter, 63762 Grossostheim (DE); BAUER, Martin, 65817 Eppstein (DE)
(74) Vertreter: Paczkowski, Marcus

(56) Entgegenhaltungen:
- EP-A1- 0 614 881
- EP-A1- 0 868 849
- WO-A1-93/09669
- DE-A1- 1 542 964
- HAMAMOTO H ET AL: "Composition for injection of medicine slightly soluble in water contains meglumine, and fatty acid", WPI / 2017 CLARIVATE ANALYTICS,, Bd. 2010, Nr. 16, 4. August 2010 (2010-08-04), XP002765314, -& JP 2010 037252 A (MEDOREKKUSU KK) 18. Februar 2010 (2010-02-18)
- M. L. DOUGLASS ET AL: "The chemistry of nitrosamine formation, inhibition and destruction", J.SOC. COSMET. CHEM., Bd. 29, 1. September 1978 (1978-09-01), Seiten 581-606, XP55024100, USA

## Beschreibung

Die Erfindung betrifft organische Ammoniumsalze von anionischen Pestiziden, ein Verfahren zu deren Herstellung, agrochemische Zusammensetzungen, welche diese Salze enthalten, sowie Verfahren zur Bekämpfung von Schadorganismen unter Verwendung der genannten Salze und Zusammensetzungen.

Pestizide (vor allem Herbizide, Fungizide und Insektizide) sind chemische Substanzen synthetisch hergestellt oder natürlichen Ursprungs, die in Pflanzenzellen, -gewebe oder in parasitäre Organismen in oder auf der Pflanze eindringen und diese schädigen und/oder zerstören. Den größten Anteil an Pestiziden stellen Herbizide dar. Pestizide werden üblicherweise in Form von flüssigen oder festen konzentrierten Zubereitungen (Formulierungen) in der Landwirtschaft eingesetzt. Diese erleichtern dem Anwender so die Handhabung oder sorgen für eine höhere Wirksamkeit des Wirkstoffs. Die Formulierungen werden üblicherweise vor dem Einsatz mit Wasser verdünnt und anschließend durch Sprühapplikation ausgebracht.

Wasserlösliche Konzentrate (Soluble Liquids, abgekürzt mit SL) sind eine wichtige Form der Pestizidzubereitungen. Sie spielen insbesondere bei Herbiziden eine große Rolle, wobei die Pestizide oftmals als wasserlösliche Salze, die durch Neutralisation der Säureform der Herbizide mit geeigneten Basen in ihre Alkalioder Ammoniumsalze überführt werden, eingesetzt werden. Unter Umständen ist ein zweiter nicht wasserlöslicher Wirkstoff in der Pestizidzubereitung enthalten. Dann handelt es sich um ein Suspensionskonzentrat (SC), auch wenn in der wässrigen Phase ein Wirkstoff gelöst ist.

Eine besonders wichtige Rolle spielen die wasserlöslichen Salze von Herbiziden, beispielsweise des Glyphosats, Glufosinats oder der Auxin-Herbizide wie Clodinafop, 2,4-D, MCPA oder Quinclorac. Sie werden vorzugsweise als Alkalimetallsalz oder in Form verschiedener Ammoniumsalze bzw. als Gemisch dieser Salze meistens als wässrige Formulierungen verwendet.

Bei der Anwendung von Pestiziden ist es von Vorteil, wenn diese eine geringe Flüchtigkeit aufweisen, da eine hohe Flüchtigkeit insbesondere mit einem verstärkten Verwehen (Drift) kleinerer Spritztröpfchen unter 150mm Tropfendurchmesser der Pestizide mit hohen Verlusten und Eintrag in die Nichtzielvegetation beim Sprühen verbunden ist. Ein solches Verwehen ist aus ökologischen und ökonomischen Gründen unerwünscht, da unbeabsichtigte Schäden verursacht werden könnten und die Wirkung der Pestizide auf die Zielorganismen herabgesetzt wird.

Um diesen Effekt zu vermeiden, ist es bekannt, flüchtige Pestizide, die in Form freier Säuren vorliegen, als Salze einzusetzen.

US 4,405,531 und WO 97/24931 offenbaren verschiedene organische Salze von Glyphosate mit Di- und Polyaminen.

In US 5,221,791 sind Aminoalkylpyrrolidon-Salze von Pestiziden wie Dicamba beschrieben.

EP-A 0 375 624 offenbart schwerflüchtige Salze von Pestiziden mit verschiedenen Polyaminen.

In der EP-A 0 183 384 sind schwerflüchtige Salze von Dicamba mit Aminoalkoholen beschrieben.

WO 93/09669 A1 beschreibt die Verwendung einer Zusammensetzung enthaltend das Ammoniumsalz einer Fettsäure, wie z. B. Perlargonsäure, als Herbizid, wobei die Ammoniumkomponente auf einem aliphatischen Amin basiert. In DE 15 42 964 A1 wird die Herstellung von N-Methylglucaminsalzen von bestimmten Pestiziden beschrieben, um eine verbesserte Löslichkeit der Pestizide zu erreichen.

Obwohl mit den bekannten Systemen bereits gute Ergebnisse erzielt werden, bleibt doch ein breiter Raum für Verbesserungen, insbesondere im Hinblick auf eine Erhöhung der Wasserlöslichkeit der Wirkstoffe im Konzentrat, eine geringere Volatilität der Wirkstoffe und eine verbesserte Wirksamkeit durch bessere Aufnahme.

Es wurde nun gefunden, dass sich die Salze von Mono- und Dialkylglucaminen mit anionischen Pestiziden in besonderer Weise zum Einsatz als wässrige Spritzapplikationen eignen.

Gegenstand der Erfindung ist daher ein organisches Ammoniumsalz der Formel (I), 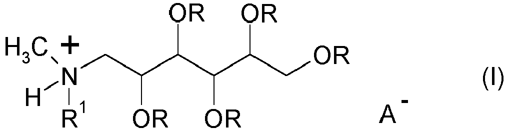 wobei die Symbole folgende Bedeutungen haben:
- (A)⁻: ist ein anionisches Pestizid,
- R¹: ist C₁-C₄-Alkyl, CH₂CH₂OH oder CH₂CH(CH₃)OH;
- R: ist H, CH₃ oder zwei benachbarte Reste R bilden zusammen eine Gruppe -C(R')₂- und
- R': ist gleich oder verschieden H oder CH₃,
dadurch gekennzeichnet, dass A ausgewählt ist aus Octansäure, Pelargonsäure und Ölsäure.

Weiterhin Gegenstand der Erfindung ist ein Verfahren zur Herstellung eines erfindungsgemäßen Ammoniumsalzes, wobei man die protonierte Form eines anionischen Pestizids (A)⁻ mit einem Glucamin der Formel (II) umsetzt, 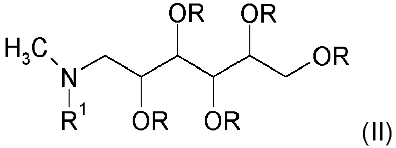 worin die Symbole die in der Formel (I) angegebenen Bedeutungen haben.

Weiterhin Gegenstand der Erfindung ist die Verwendung der erfindungsgemäßen Ammoniumsalze (I) als Pestizide.

Ebenso Gegenstand der Erfindung ist eine Pestizidzusammensetzung, enthaltend
a) ein oder mehrere erfindungsgemäße Ammoniumsalze (I) und
b) einen oder mehrere Formulierungshilfsstoffe.

Ebenso Gegenstand der Erfindung ist ein Verfahren zur Bekämpfung von Schadorganismen, wobei man den Schadorganismus oder dessen Lebensraum mit einem erfindungsgemäßen Ammoniumsalz (I) oder einer erfindungsgemäßen Pestizidzusammensetzung in Kontakt bringt.

Weiterhin Gegenstand der Erfindung ist die Verwendung eines erfindungsgemäßen Ammoniumsalzes (I) zur Verminderung der Flüchtigkeit des als Salz vorliegenden anionischen Pestizids.

Die erfindungsgemäßen Ammoniumsalze (I) eignen sich in hervorragender Weise zum Einsatz als wässrige Spritzapplikationen und zeigen insbesondere eine gute Wasserlöslichkeit und verminderte Flüchtigkeit bei nicht erhöhter Drift in der Anwendung.

Das Kation der erfindungsgemäßen Ammoniumsalze (I) entsteht durch Protonierung eines Glucamins (II), 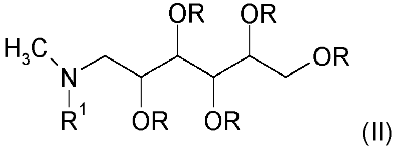 wobei die Symbole folgende Bedeutungen haben:
- R¹: ist C₁-C₄-Alkyl, CH₂CH₂OH oder CH₂CH(CH₃)OH;
- R: ist H, CH₃ oder zwei benachbarte Reste R bilden zusammen eine Gruppe -C(R')₂- und
- R': ist gleich oder verschieden H oder CH₃.

Bevorzugt haben die Symbole in den Formeln (I) und (II) folgende Bedeutungen:
- R¹: ist bevorzugt C₁-C₄-Alkyl oder CH₂CH₂OH.
- R: ist bevorzugt H oder CH₃.

Bevorzugt sind Kationen, in denen alle Symbole die bevorzugten Bedeutungen haben.

Besonders bevorzugt haben die Symbole in den Formeln (I) und (II) folgende Bedeutungen:
- R¹: ist besonders bevorzugt CH₃ oder CH₂CH₂OH.
- R: ist besonders bevorzugt H.

Besonders bevorzugt sind Kationen, in denen alle Symbole die besonders bevorzugten Bedeutungen haben.

Ganz besonders bevorzugt sind protoniertes Dimethylglucamin (R¹ = CH₃, R = H) und Hydroxyethylglucamin (R¹ = CH₂CH₂OH, R = H).

Der Pentahydroxyhexylrest in den Alkylglucaminen der Formel (I) und (II) verfügt über verschiedene chirale Zentren, so dass jeweils mehrere Stereoisomere existieren können. Üblicherweise werden die Alkylglucamine der Formel (II) aus natürlich vorkommenden Zuckern, wie der D-Glucose hergestellt, grundsätzlich ist aber auch die Verwendung anderer natürlicher oder synthetischer Hexosen oder anderer C₆-Bausteine möglich, so dass unterschiedliche Stereoisomere der Formeln (I) und (II) resultieren können.

Die Herstellung der Alkylglucamine der Formel (II) ist hinlänglich vorbeschrieben und dem Fachmann bekannt. Sie erfolgt (für Verbindungen mit R¹ = C₁-C₄-Alkyl) beispielsweise wie in der EP-A 1 676 831 beschrieben durch reduktive Alkylierung von N-Alkylpolyhydroxylaminen mit Aldehyden oder Ketonen in Gegenwart von Wasserstoff und eines Übergangsmetallkatalysators.

Dimethylglucamin kann beispielsweise gemäß EP-A 0 614 881 hergestellt werden. Dimethylglucamin ist als 50 % wässrige Lösung einsetzbar. Hydroxyethyl-N-Methyl-glucamin kann durch Reaktion von N-methylglucamin mit Ethylenoxid in wässriger Lösung hergestellt werden. Dimethylglucamin und Hydroxyethyl-N-Methyl-glucamin sind als tertiäre Amine wenig anfällig für die Bildung von Nitrosaminen und dementsprechend bevorzugt.

Verbindungen, in denen zwei benachbarte Reste R zusammen eine Gruppe -C(R')₋ bilden, können durch Bildung des entsprechenden Acetonids oder Acetals mit Aceton beziehungsweise Acetaldehyd oder Formaldehyd erhalten werden.

Die Alkylglucamine der Formel (II) basieren bevorzugt auf nachwachsenden Rohstoffen und zeichnen sich durch ein vorteilhaftes toxikologisches und ökologisches Profil aus.

Die Gruppe (A)⁻ in der Formel (I) steht für ein anionisches Pestizid, wobei A ausgewählt ist aus Octansäure, Pelargonsäure und Ölsäure.

Das anionische Pestizid die konjugierte Base einer der vorstehend genannten pestiziden Bremsted Säuren, die einen pKₐ-Wert von 1,5 bis 7, bevorzugt 2 bis 6 und besonders bevorzugt 2,5 bis 5,5 aufweisen.

Die erfindungsgemäßen Ammoniumsalze (I) zeigen im Wesentlichen dasselbe Wirkspektrum wie die entsprechenden Pestizide A und können somit gegen dieselben, dem Fachmann bekannten Zielorganismen eingesetzt werden.

Mit den erfindungsgemäßen Ammoniumsalzen (I) lassen sich erfindungsgemäße Pestizidzusammensetzungen, insbesondere wässrige Herbizid-Zusammensetzungen herstellen, mit ausgezeichneter Wirkung und ausgezeichneten anwendungstechnischen Eigenschaften, wie der verringerten Flüchtigkeit ohne veränderte Drift.

Die Pestizidzusammensetzungen enthalten
a) ein oder mehrere erfindungsgemäße Ammoniumsalze (I), wie oben beschrieben, und
b) einen oder mehrere Formulierungshilfsstoffe.

In einer bevorzugten Ausführungsform der Erfindung beträgt die Menge des einen oder der mehreren Ammoniumsalze (I) a) in den erfindungsgemäßen Zusammensetzungen mehr als 100 g/l, bevorzugt mehr als 200 g/l und besonders bevorzugt mehr als 300 g/l. Diese Mengenangaben beziehen sich auf das Gesamtgewicht der erfindungsgemäßen Pestizidzusammensetzung und auf die Menge an freier Säure (d.h. der protonierten Form), dem sogenannten Säureäquivalent ("acid equivalent", a.e.) der anionischen Pestizide A.

Bei den Formulierungshilfsstoffen (b) handelt es sich beispielsweise um Lösungsmittel, Tenside, Entschäumer, funktionelle Polymere, Adjuvants, Frostschutzmittel, Konservierungsmittel, Farbstoffe und/oder Ammoniumsalze.

Bevorzugt als Lösungsmittel ist Wasser. Bevorzugt ist auch der Einsatz von einem oder mehreren Cosolventien. Bei den Cosolventien kann es sich um ein einziges Lösemittel oder ein Gemisch zweier oder mehrerer Lösemittel handeln. Dazu eignen sich alle polaren Lösemittel, die mit der wässrigen Pestizidzusammensetzung kompatibel sind und eine homogene Phase bilden. Geeignete Cosolventien sind beispielsweise einwertige Alkohole, wie Methanol, Ethanol, Propanole, Butanole, Benzylalkohol oder weitere mehrwertige Alkohole wie Ethylenglykol, Diethylenglykol, Propylenglykol, Dipropylenglykol, Glycerin oder Polyglykole wie Polyethylen-, Polypropylen- oder gemischte Polyalkylenglykole (PAGs). Weitere geeignete Lösemittel sind Ether wie beispielsweise Propylenglykolmono- oder dimethylether, Dipropylenglykolmono- oder dimethylether, Amide wie beispielsweise N-Methyl- oder N-Ethylpyrrolidon, Milchsäure-, Capryl- oder Decansäuredimethylamid.

Bei den Tensiden kann es sich generell um alle mit der Zusammensetzung kompatiblen nichtionischen, amphoteren, kationischen oder anionische Tenside handeln.

Beispiele für nicht-ionische Tenside sind Glucamide, insbesondere wie in der WO 2014/067663 beschrieben, Ethoxylate und Alkoxylate von längerkettigen aliphastischen oder aromatischen Alkoholen, Fettaminethoxylate, längerkettige Etheraminalkoxylate, (gegebenenfalls ethoxylierte) Sorbitanester, Alkylpolyglycoside. Geeignete amphotere Tenside sind u.a. langkettige Alkyldimethylbetaine oder Alkyldimethylaminoxide, oder Alkyldimethylaminamidopropylaminoxide. Unter den anionischen Tensiden sind beispielsweise Ethersulfate von ethoxylierten Fettalkoholen, Umsetzungsprodukte von (gegebenenfalls ethoxylierten) langkettigen Alkoholen mit Phosphorsäurederivaten geeignet. Unter langkettig werden lineare oder verzweigte Kohlenwasserstoffketten mit mindestens 6 und maximal 22 Kohlenstoffatomen geeignet.

Als Entschäumer eignen sich Fettsäurealkylesteralkoxylate, Organopolysiloxane wie Polydimethylsiloxane und deren Gemische mit mikrofeiner, gegebenenfalls silanierter Kieselsäure; Perfluoralkylphosphonate und -phosphinate, Paraffine, Wachse und Mikrokristallinwachse und deren Gemische mit silanierter Kieselsäure. Vorteilhaft sind auch Gemische verschiedener Schauminhibitoren, beispielsweise solche aus Silikonöl, Paraffinöl und/oder Wachsen. Bevorzugt sind die in der deutschen Patentanmeldung DE 10 2014 208 244.7 beschriebenen Entschäumer.

Bei den funktionellen Polymeren, die in der erfindungsgemäßen PestizidZusammensetzung enthalten sein können, handelt es sich hochmolekulare Verbindungen synthetischen oder natürlichen Ursprungs mit einer Molmasse von größer als 10.000. Die funktionellen Polymere können beispielsweise als Antidrift-Agent wirken oder die Regenfestigkeit steigern.

In einer weiteren Ausführungsform der Erfindung enthalten die erfindungsgemäßen Pestizidzusammensetzungen ein oder mehrere Adjuvantien, wie sie bekanntermaßen in wässrigen Pestizidzusammensetzungen verwendet werden können.

Bevorzugt sind dies Fettaminethoxylate, Etheraminethoxylate, Alkylbetaine oder Amidoalkylbetaine, Aminoxide oder Amidoalkylaminoxide, Alkylpolylglycoside oder Copolymere aus Glycerin, Kokosfettsäure und Phthalsäure.

Diese Adjuvantien sind aus der Literatur bekannt und beispielsweise in der WO 2009/029561 beschrieben.

Weiterhin bevorzugt als Adjuvantien sind Glucamide, insbesondere wie in der WO 2014/067663 beschrieben.

Ebenso bevorzugt als Adjuvantien sind driftreduzierende Polyglycerinester wie in der WO 2014/063818 beschrieben, welche ein oder mehrere Copolymere A) enthalten, wobei die Copolymere eine oder mehrere Struktureinheiten, hervorgegangen aus
a) 19,9 bis 75,9 Gew.-% Glycerin
b) 0,1 bis 30 Gew.-% mindestens einer Dicarbonsäure und
c) 24 bis 80 Gew.-% mindestens einer Monocarbonsäure gemäß Formel (III),

   R²-COOH (III)

   wobei R² (C₅-C₂₉)-Alkyl; (C₇-C₂₉)-Alkenyl; Phenyl oder Naphthyl darstellt, enthalten.

Als Konservierungsmittel können organische Säuren und ihre Ester, beispielsweise Ascorbinsäure, Ascorbinpalmitat, Sorbat, Benzoesäure, Methylund Propyl-4-hydroxybenzoat, Propionate, Phenol, beispielsweise 2-Phenylphenat, 1,2 Benzisothiazolin-3-on, Formaldehyd, schwefelige Säure und deren Salze eingesetzt werden.

Als Frostschutzmittel eignen sich beispielsweise Ethylenglykol, Propylenglykol, Glycerin und Harnstoff

Als Ammoniumsalze eignen sich wasserlösliche Ammoniumsalze wie Ammoniumsulfat, Ammoniumnitrat, Ammoniumnitratharnstoff, Ammoniumphosphat, Ammoniumcitrat, Ammoniumthiosulfat und/oder Ammoniumchlorid, bevorzugt Ammoniumsulfat, Ammoniumnitrat, Ammoniumcitrat und/oder Ammoniumnitratharnstoff, besonders bevorzugt Ammoniumsulfat.

Neben den Ammoniumsalzen (I) a) und Formulierungshilfsstoffen b) können die Pestizidzusammensetzungen weitere Pestizide c) enthalten.

Im Folgenden werden Beispiele für Pestizide c) genannt, die Kombinationspartner der Ammoniumsalze (I) bilden können.

### Als Beispiele für Herbizide seien genannt:

Wirkstoffe, die auf einer Inhibition von beispielsweise Acetolaktat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441 445 oder "The Pesticide Manual", 16th edition, The British Crop Protection Council and the Royal Soc. of Chemistry, 2012 und dort zitierter Literatur beschrieben sind. Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:
Acetochlor, Acibenzolar, Acibenzolar-S-methyl, Aclonifen, Alachlor, Allidochlor, Alloxydim, Alloxydim-sodium, Ametryn, Amicarbazone, Amidochlor, Amidosulfuron, Aminocyclopyrachlorkalium, Aminocyclopyrachlormethyl, Ammoniumsulfamat, Ancymidol, Anilofos, Atrazine, Aviglycin, Azafenidin, Azimsulfuron, Aziprotryn, Beflubutamid, Benazolinethyl, Bencarbazone, Benfluralin, Benfuresate, Bensulide, Benzfendizone, Benzobicyclon, Benzofenap, Benzofluor, Benzoylprop, Benzyladenin, Bromobutide, Bromofenoxim, Bromuron, Buminafos, Busoxinone, Butachlor, Butafenacil, Butamifos, Butenachlor, Butralin, Butroxydim, Butylate, Cafenstrole, Carbaryl, Carbetamide, Carvone, Chlorcholinchlorid, Chlomethoxyfen, Chlorbromuron, Chlorbufam, Chlorfenac, Chlorfenacnatrium, Chlorfenprop, Chlorflurenol-methyl, Chloridazon, Chlorimuronethyl, Chlormequatchlorid, Chlornitrofen, 4-Chlorophenoxyacetic acid, Chlorophthalim, Chlorpropham, Chlorotoluron, Chlorsulfuron, Cinidon, Cinidonethyl, Cinmethylin, Cinosulfuron, Clethodim, Clodinafoppropargyl, Clofencet, Clomazone, Clomeprop, Cloprop, Cloransulam, Cloransulammethyl, Cloxyfonac, Cumyluron, Cyanamide, Cyanazine, Cyclanilide, Cycloate, Cyclosulfamuron, Cycloxydim, Cycluron, Cyperquat, Cyprazine, Cyprazole, Cytokinine, Daimuron/Dymron, Daminozide, Dazomet, n-Decanol, Desmedipham, Desmetryn, Detosyl-Pyrazolate (DTP), Diallate, Diaminozid, Dichlobenil, Diclosulam, Diethatyl, Diethatylethyl, Difenoxuron, Diflufenican, Diflufenzopyrnatrium, Dikegulac-sodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Diisopropylnaphthalene, Dipropetryn, Dithiopyr, Diuron, DNOC, Eglinazineethyl, EPTC, Esprocarb, Ethalfluralin, Ethametsulfuron, Ethametsulfuronmethyl, Ethylnaphthylacetat, Ethephon, Ethidimuron, Ethiozin, Ethofumesate, Ethoxyfen, Ethoxyfenethyl, Ethoxysulfuron, Etobenzanid, F-5331, d.h. N-[2-Chlor-4-fluor-5-[4-(3-fluorpropyl)-4,5-dihydro-5-oxo-1H-tetrazol-1-yl]-phenyl]-ethansulfonamid, F-7967, d. h. 3-[7-Chlor-5-fluor-2-(trifluormethyl)-1H-benzimidazol-4-yl]-1-methyl-6-(trifluormethyl) pyrimidin-2,4(1H,3H)-dion, Fenoxasulfone, Fentrazamide, Fenuron, Flazasulfuron, Fluazolate, Flucarbazonesodium, Flucetosulfuron, Fluchloralin, Flufenacet (Thiafluamide), Flumetralin, Flumetsulam, Flumioxazin, Flumipropyn, Fluometuron, Fluorodifen, Flupoxam, Flupropacil, Flupyrsulfuron, Flupyrsulfuronmethylsodium, Fluridone, Flurochloridone, Flurprimidol, Flurtamone, Fluthiacetmethyl, Fluthiamide, Forchlorfenuron, Furyloxyfen, Gibberellinsäure, H-9201, d. h. O-(2,4-Dimethyl-6-nitrophenyl)-O-ethylisopropylphosphor amidothioat, Halosafen, Halosulfuronmethyl, Hexazinone, HW-02, d. h. 1-(Dimethoxyphosphoryl)-ethyl-(2,4-dichlorphenoxy)acetat, Imazosulfuron, Inabenfide, Indanofan, Indaziflam, lodosulfuron, lodosulfuronmethyl-natrium, lofensulfuron, lofensulfuronnatrium, loxynil, Ipfencarbazone, Isocarbamid, Isopropalin, Isoproturon, Isouron, Isoxaben, Isoxachlortole, Isoxaflutole, KUH-043, d. h. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, Karbutilate, Ketospiradox, Lenacil, Linuron, Maleinsäurehydrazid, Mefenacet, Mefluidide, Mepiquat-chlorid, Methabenzthiazuron, Metam, Metamitron, Metazachlor, Metazasulfuron, Methazole, Methiopyrsulfuron, Methiozolin, Methoxyphenone, Methyldymron, 1-Methylcyclopropen, Methylisothiocyanat, Metobenzuron, Metobromuron, Metolachlor, S-Metolachlor, Metosulam, Metoxuron, Metribuzin, Metsulfuron, Metsulfuronmethyl, Molinate, Monalide, Monocarbamide, Monocarbamidedihydrogensulfat, Monolinuron, Monosulfuron, Monosulfuronester, Monuron, MT-128, d. h. 6-Chlor-N-[(2E)-3-chlorprop-2-en-1-yl]-5-methyl-N-phenylpyridazin-3-amin, MT-5950, d. h. N-[3-Chlor-4-(1-methylethyl)-phenyl]-2-methylpentanamid, NGGC-011, Naproanilide, Napropamide, NC-310, d.h. 4-(2,4-Dichlorobenzoyl)-1-methyl-5-benzyloxypyrazole, Neburon, Nipyraclofen, Nitralin, Nitrofen, Nitroguaiacolate, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquatdichlorid, Pendimethalin, Pendralin, Penoxsulam, Pentanochlor, Pentoxazone, Perfluidone, Pethoxamid, Phenisopham, Phenmedipham, Phenmediphamethyl, Picolinafen, Pinoxaden, Piperophos, Pirifenop, Pirifenopbutyl, Pretilachlor, Probenazole, Profluazol, Procyazine, Prodiamine, Prifluraline, Profoxydim, Prohexadionecalcium, Prohydrojasmone, Prometon, Prometryn, Propachlor, Propanil, Propazine, Propham, Propisochlor, Propyrisulfuron, Propyzamide, Prosulfalin, Prosulfocarb, Prosulfuron, Prynachlor, Pyraclonil, Pyrasulfotole, Pyrazolynate (Pyrazolate), Pyrazosulfuron, Pyrazosulfuronethyl, Pyrazoxyfen, Pyribambenz, Pyribambenz-isopropyl, Pyribambenzpropyl, Pyribenzoxim, Pyributicarb, Pyridafol, Pyridate, Pyriftalid, Pyrimisulfan, Pyroxasulfone, Pyroxsulam, Quinoclamine, Rimsulfuron, Saflufenacil, Secbumeton, Sethoxydim, Siduron, Simazine, Simetryn, SN-106279, d. h. Methyl-(2R)-2-({7-[2-chlor-4-(trifluormethyl)phenoxy]-2-naphthyl}oxy)propanoat, Sulfallate (CDEC), Sulfentrazone, Sulfo-sulfuron, SW-065, SYN-523, SYP-249, d.h. 1-Ethoxy-3-methyl-1-oxobut-3-en-2-yl-5-[2-chlor-4-(trifluormethyl)phenoxy]-2-nitrobenzoat, SYP-300, d.h. 1-[7-Fluor-3-oxo-4-(prop-2-in-1-yl)-3,4-dihydro-2H-1,4-benzoxazin-6-yl]-3-propyl-2-thioxoimidazolidin-4,5-dion, Tebutam, Tebuthiuron, Tecnazene, Tefuryltrione, Tepraloxydim, Terbacil, Terbucarb, Terbuchlor, Terbumeton, Terbuthylazine, Terbutryn, Thenylchlor, Thiafluamide, Thiazafluron, Thiazopyr, Thidiazimin, Thidiazuron, Thiencarbazonemethyl, Thifensulfuron, Thifensulfuronmethyl, Thiobencarb, Tiocarbazil, Tralkoxydim, Triafamone, Triallate, Triasulfuron, Triaziflam, Triazofenamide, Tribenuron, Tribenuronmethyl, Tribufos, Tridiphane, Trietazine, Trifloxysulfuron, Trifloxysulfuronnatrium, Trifluralin, Triflusulfuron, Triflusulfuronmethyl, Trimeturon, Trinexapacethyl, Tritosulfuron, Tsitodef, Uniconazole, Uniconazole-P, Vernolate, ZJ-0862, d. h. 3,4-Dichlor-N-{2-[(4,6-dimethoxypyrimidin-2-yl)oxy]benzyl}anilin.

Als Beispiele für Pflanzenwuchsregulatoren seien ferner natürliche Pflanzenhormone wie Ester der Salicylsäure, Kinetin und Brassinosteroide genannt.

Weiter seien Substanzen genannt, die als Pflanzenwuchsregulatoren und/oder Pflanzenstärkungsmittel wirken können, um den Einfluss von Stressfaktoren wie Hitze, Kälte, Trockenheit, Salz, Sauerstoffmangel bzw.- Überschwemmung auf das Pflanzenwachstum zu mindern. Hier seien beispielhaft genannt Glycinbetain (Betain), Cholin, Kaliumphosphat oder andere Phosphatsalze, sowie Silikate.

Als Beispiele für Pflanzennährstoffe seien übliche anorganische oder organische Dünger zur Versorgung von Pflanzen mit Makro- und/oder Mikronährstoffen genannt.

### Als Beispiele für Fungizide seien genannt:

(1) Inhibitoren der Ergosterol-Biosynthese, wie beispielsweise Aldimorph, Azaconazol, Bitertanol, Bromuconazol, Cyproconazol, Diclobutrazol, Difenoconazol, Diniconazol, Diniconazol-M, Dode-morph, Dodemorph Acetat, Epoxiconazol, Etaconazol, Fenarimol, Fenbuconazol, Fenhexamid, Fenpropidin, Fenpropimorph, Fluquinconazol, Flurprimidol, Flusilazol, Flutriafol, Furconazol, Furconazol-Cis, Hexaconazol, Imazalil, Imazalil Sulfat, Imibenconazol, Ipconazol, Metconazol, Myclobutanil, Naftifin, Nuarimol, Oxpoconazol, Paclobutrazol, Pefurazoat, Penconazol, Piperalin, Prochloraz, Propiconazol, Prothioconazol, Pyributicarb, Pyrifenox, Quinconazol, Simeconazol, Spiroxamin, Tebuconazol, Terbinafin, Tetraconazol, Triadimefon, Triadimenol, Tridemorph, Triflumizol, Triforin, Triticonazol, Uniconazol, Uniconazol-p, Viniconazol, Voriconazol, 1-(4-Chlorphenyl)-2-(1H-1,2,4-triazol-1-yl)cycloheptanol, Methyl-1-(2,2-dimethyl-2,3-dihydro-1H-inden-1-yl)-1H-imidazol-5-carboxylat, N'-{5-(Difluormethyl)-2-methyl-4-[3-(trimethylsilyl)propoxy]phenyl}-N-ethyl-N-methylimidoformamid, N-Ethyl-N-methyl-N'-{2-methyl-5-(trifluormethyl)-4-[3-(trimethylsilyl)propoxy]phenyl}imidoformamid und O-[1-(4-Methoxyphenoxy)-3,3-dimethylbutan-2-yl]-1H-imidazol-1-carbothioat.
(2) Inhibitoren der Respiration (Atmungsketten-Inhibitoren), wie beispielsweise Bixafen, Boscalid, Carboxin, Diflumetorim, Fenfuram, Fluopyram, Flutolanil, Fluxapyroxad, Furametpyr, Furmecyclox, Isopyrazam Mischung des synepimeren Razemates 1RS,4SR,9RS und des antiempimeren Razemates 1RS,4SR,9SR, Isopyrazam (anti-epimeres Razemat), Isopyrazam (anti-epimeres Enantiomer 1R,4S,9S), Isopyrazam (anti-epimeres Enantiomer 1S,4R,9R), Isopyrazam (synepimeres Razemat 1RS,4SR,9RS), Isopyrazam (synepimeres Enantiomer 1R,4S,9R), Iso-pyrazam (syn-epimeres Enantiomer 1S,4R,9S), Mepronil, Oxycarboxin, Penflufen, Penthiopyrad, Sedaxane, Thifluzamid, 1-Methyl-N-[2-(1,1,2,2-tetrafluorethoxy)phenyl]-3-(trifluormethyl)-1H-pyrazol-4-carboxamid, 3-(Difluormethyl)-1-methyl-N-[2-(1,1,2,2-tetrafluorethoxy)phenyl]-1H-pyrazol-4-carboxamid, 3-(Difluormethyl)-N-[4-fluor-2-(1,1,2,3,3,3-hexafluorpropoxy)phenyl]-1-methyl-1H-pyrazol-4-carboxamid, N-[1-(2,4-Dichlorphenyl)-1-methoxypropan-2-yl]-3-(difluormethyl)-1-methyl-1H-pyrazol-4-carboxamid, 5,8-Difluor-N-[2-(2-fluor-4-{[4-(trifluormethyl)pyridin-2-yl]oxy}phenyl)ethyl]quinazolin-4-amin, N-[9-(Dichlormethylen)-1,2,3,4-tetrahydro-1,4-methanonaphthalen-5-yl]-3-(difluormethyl)-1-methyl-1H-pyrazol-4-carboxamid, N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid und N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid.
(3) Inhibitoren der Respiration (Atmungsketten-Inhibitoren) am Komplex III der Atumungskette, wie beispielsweise Ametoctradin, Amisulbrom, Azoxystrobin, Cyazofamid, Coumethoxystrobin, Coumoxystrobin, Dimoxystrobin, Enestroburin, Famoxadon, Fenamidon, Fenoxystrobin, Fluoxastrobin, Kresoxim-Methyl, Metominostrobin, Orysastrobin, Picoxystrobin, Pyraclostrobin, Pyrametostrobin, Pyraoxystrobin, Pyribencarb, Triclopyricarb, Trifloxystrobin, (2E)-2-(2-{[6-(3-Chlor-2-methylphenoxy)-5-fluorpyrimidin-4-yl]oxy}phenyl)-2-(methoxyimino)-N-methylethanamid, (2E)-2-(Methoxyimino)-N-methyl-2-(2-{[({(1E)-1-[3-(trifluormethyl)phenyl]ethyliden}amino)oxy]methyl}phenyl)ethanamid, (2E)-2-(Methoxyimino)-N-methyl-2-{2-[(E)-({1-[3-(trifluormethyl)phenyl]ethoxy}imino)methyl]phenyl}ethanamid, (2E)-2-{2-[({[(1E)-1-(3-{[(E)-1-Fluor-2-phenylethenyl]oxy}phenyl)ethyliden]-amino}oxy)methyl]phenyl}-2-(methoxyimino)-N-methylethanamid, (2E)-2-{2-[({[(2E,3E)-4-(2,6-Dichlorphenyl)but-3-en-2-yliden]amino}oxy)methyl]phenyl}-2-(methoxyimino)-N-methylethanamid, 2-Chlor-N-(1,1,3-trimethyl-2,3-dihydro-1H-inden-4-yl)pyridin-3-carboxamid, 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, Methyl-(2E)-2-{2-[({cyclopropyl[(4-methoxyphenyl)imi-no]methyl}sulfanyl)methyl]phenyl}-3-methoxyprop-2-enoat, N-(3-Ethyl-3,5,5-trimethylcyclohexyl)-3-(formylamino)-2-hydroxybenzamid, 2-{2-[(2,5-Dimethyl-phenoxy)methyl]phenyl}-2-methoxy-N-methylacetamid und (2R)-2-{2-[(2,5-Dimethylphenoxy)methyl]phenyl}-2-methoxy-N-methylacetamid.
(4) Inhibitoren der Mitose und Zellteilung, wie beispielsweise Benomyl, Carbendazim, Chlorfe-nazol, Diethofencarb, Ethaboxam, Fluopicolid, Fuberidazol, Pencycuron, Thiabendazol, Thi-ophanat-Methyl, Thiophanat, Zoxamid, 5-Chlor-7-(4-methylpiperidin-1-yl)-6-(2,4,6-trifluorphenyl)[1,2,4]triazolo[1,5-a]pyrimidin und 3-Chlor-5-(6-chlorpyridin-3-yl)-6-methyl-4-(2,4,6-trifluorphenyl)pyridazin.
(5) Verbindungen mit Multisite-Aktivität, wie beispielsweise Bordeauxmischung, Captafol, Captan, Chlorothalonil, Kupferzubereitungen wie Kupferhydroxid, Kupfernaphthenat, Kupferoxid, Kupferoxychlorid, Kupfersulfat, Dichlofluanid, Dithianon, Dodine, Dodine freie Base, Ferbam, Fluorofolpet, Folpet, Guazatin, Guazatinacetat, Iminoctadin, Iminoctadinalbesilat, Iminoctadintriacetat, Mankupfer, Mancozeb, Maneb, Metiram, Zinkmetiram, Kupfer-Oxin, Propamidin, Propineb, Schwefel und Schwefelzubereitungen wie beispielsweise Calciumpolysulfid, Thiram, Tolylfluanid, Zineb und Ziram.
(6) Resistenzinduktoren, wie beispielsweise Acibenzolar-S-Methyl, Isotianil, Probenazol und Tiadinil.
(7) Inhibitoren der Aminosäure- und Protein-Biosynthese, wie beispielsweise Andoprim, Blasti-cidin-S, Cyprodinil, Kasugamycin, Kasugamycin Hydrochlorid Hydrat, Mepanipyrim, Pyrimethanil und 3-(5-Fluor-3,3,4,4-tetramethyl-3,4-dihydroisoquinolin-1-yl)quinolin.
(8) Inhibitoren der ATP Produktion, wie beispielsweise Fentin Acetat, Fentin Chlorid, Fentin Hydroxid und Silthiofam.
(9) Inhibitoren der Zellwandsynthese, wie beispielsweise Benthiavalicarb, Dimethomorph, Flu-morph, Iprovalicarb, Mandipropamid, Validamycin A und Valifenalat.
(10) Inhibitoren der Lipid- und Membran-Synthese, wie beispielsweise Biphenyl, Chloroneb, Dicloran, Edifenphos, Etridiazol, lodocarb, Iprobenfos, Isoprothiolan, Propamocarb, Propamocarb Hydrochlorid, Prothiocarb, Pyrazophos, Quintozen, Tecnazene und Tolclofos-Methyl.
(11) Inhibitoren der Melanin-Biosynthese, wie beispielsweise Carpropamid, Diclocymet, Fenoxanil, Fthalid, Pyroquilon, Tricyclazol und 2,2,2-Trifluorethyl {3-methyl-1-[(4-methylbenzoyl)amino]butan-2-yl}carbamat.
(12) Inhibitoren der Nukleinsäuresynthese, wie beispielsweise Benalaxyl, Benalaxyl-M (Kirala-xyl), Bupirimat, Clozylacon, Dimethirimol, Ethirimol, Furalaxyl, Hymexazol, Metalaxyl, Metalaxyl-M (Mefenoxam), Ofurace, Oxadixyl und Oxolinsäure.
(13) Inhibitoren der Signaltransduktion, wie beispielsweise Chlozolinat, Fenpiclonil, Fludioxonil, Iprodion, Procymidon, Quinoxyfen und Vinclozolin.
(14) Entkoppler, wie beispielsweise Binapacryl, Dinocap, Ferimzon, Fluazinam und Meptyldinocap.
(15) Weitere Verbindungen, wie beispielsweise Benthiazol, Bethoxazin, Capsimycin, Carvon, Chinomethionat, Pyriofenon (Chlazafenon), Cufraneb, Cyflufenamid, Cymoxanil, Cyprosulfa-mide, Dazomet, Debacarb, Dichlorophen, Diclomezin, Difenzoquat, Difenzoquat Methylsulphat, Diphenylamin, Ecomat, Fenpyrazamin, Flumetover, Fluoromid, Flusulfamid, Flutianil, Fosetyl-Aluminium, Fosetyl-Calcium, Fosetyl-Natrium, Hexachlorbenzol, Irumamycin, Methasulfocarb, Methylisothiocyanat, Metrafenon, Mildiomycin, Natamycin, Nickel Dimethyldithiocarbamat, Nitrothal-Isopropyl, Octhilinone, Oxamocarb, Oxyfenthiin, Pentachlorphenol und dessen Salze, Phenothrin, Phosphorsäure und deren Salze, Propamocarb-Fosetylat, Propanosin-Natrium, Proquinazid, Pyrimorph, (2E)-3-(4-Tert-butylphenyl)-3-(2-chlorpyridin-4-yl)-1-(morpholin-4-yl)prop-2-en-1-on, (2Z)-3-(4-Tert-butylphenyl)-3-(2-chlorpyridin-4-yl)-1-(morpholin-4-yl)prop-2-en-1-on, Pyrrolnitrin, Tebufloquin, Tecloftalam, Tolnifanid, Triazoxid, Trichlamid, Zarilamid, (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoat, 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-Methoxyphenoxy)-3,3-dimethylbutan-2-yl-1H-imidazol-1-carboxylat, 2,3,5,6-Tetrachlor-4-(methylsulfonyl)pyridin, 2,3-Dibutyl-6-chlorthieno[2,3-d]pyrimidin-4(3H)-on, 2,6-Dimethyl-1H,5H-[1,4]dithiino[2,3-c:5,6-c']dipyrrol-1,3,5,7(2H,6H)-tetron, 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, 2-Butoxy-6-iod-3-propyl-4H-chromen-4-on, 2-Chlor-5-[2-chlor-1-(2,6-difluor-4-methoxyphenyl)-4-methyl-1H-imidazol-5-yl]pyridin, 2-Phenylphenol und dessen Salze, 3-(4,4,5-Trifluor-3,3-dimethyl-3,4-dihydroisoquinolin-1-yl)quinolin, 3,4,5-Trichlorpyridin-2,6-dicarbonitril, 3-[5-(4-Chlorphenyl)-2,3-dimethyl-1,2-oxazolidin-3-yl]pyridin, 3-Chlor-5-(4-chlorphenyl)-4-(2,6-difluorphenyl)-6-methylpyridazin, 4-(4-Chlorphenyl)-5-(2,6-difluorphenyl)-3,6-dimethylpyridazin, 5-Amino-1,3,4-thiadiazol-2-thiol, 5-Chlor-N'-phenyl-N'-(prop-2-in-1-yl)thiophen-2-sulfonohydrazid, 5-Fluor-2-[(4-fluorbenzyl)oxy]pyrimidin-4-amin, 5-Fluor-2-[(4-methylbenzyl)oxy]pyrimidin-4-amin, 5-Methyl-6-octyl[1,2,4]triazolo[1,5-a]pyrimidin-7-amin, Ethyl-(2Z)-3-amino-2-cyan-3-phenylprop-2-enoat, N'-(4-{[3-(4-Chlorbenzyl)-1,2,4-thiadiazol-5-yl]oxy}-2,5-dimethylphenyl)-N-ethyl-N-methylimidoformamid, N-(4-Chlorbenzyl)-3-[3-methoxy-4-(prop-2-in-1-yloxy)phenyl]propanamid, N-[(4-Chlorphenyl)(cyan)methyl]-3-[3-methoxy-4-(prop-2-in-1-yloxy)phenyl]propanamid, N-[(5-Brom-3-chlorpyridin-2-yl)methyl]-2,4-dichlorpyridin-3-carboxamid, N-[1-(5-Brom-3-chlorpyridin-2-yl)ethyl]-2,4-dichlorpyridin-3-carboxamid, N-[1-(5-Brom-3-chlorpyridin-2-yl)ethyl]-2-fluor-4-iodpyridin-3-carboxamid, N-{(E)-[(Cyclopropylmethoxy)imino][6-(difluormethoxy)-2,3-difluorphenyl]methyl}-2-phenylacetamid, N-{(Z)-[(Cyclopropylmethoxy)-iimino][6-(difluormethoxy)-2,3-difluorphenyl]methyl}-2-phenylacetamid, N'-{4-[(3-Tert-butyl-4-cyano-1,2-thiazol-5-yl)oxy]-2-chlor-5-methylphenyl}-N-ethyl-N-methylimidoformamid, N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazol-4-carboxamid, N-Methyl-2-(1-{[5-methyl-3-(trifluormethyl)-1H-pyrazol-1-yl]acetyl}¬piperidin-4-yl)-N-[(1R)-1,2,3,4-tetrahydronaphthalen-1-yl]-1,3-thiazol-4-carboxamid, N-Methyl-2-(1-{[5-methyl-3-(trifluormethyl)-1H-pyrazol-1-yl]acetyl}piperidin-4-yl)-N-[(1S)-1,2,3,4-tetrahydronaph¬thalen-1-yl]-1,3-thiazol-4-carboxamid, Pentyl-{6-[({[(1-methyl-1H-tetrazol-5-yl)(phenyl)methyli-den]amino}oxy)methyl]pyridin-2-yl}carbamat, Phenazin-1-carbonsäure, Chinolin-8-ol, Chinolin-8-olsulfat(2:1) und Tert-butyl {6-[({[(1-methyl-1H-tetrazol-5-yl)(phenyl)methylen]amino}oxy)methyl]pyridin-2-yl}carbamat.
(16) Weitere Verbindungen, wie beispielsweise 1-Methyl-3-(trifluormethyl)-N-[2'-(trifluormethyl)biphenyl-2-yl]-1H-pyrazol-4-carboxamid, N-(4'-Chlorbiphenyl-2-yl)-3-(difluormethyl)-1-methyl-1H-pyrazol-4-carboxamid, N-(2',4'-Dichlorbiphenyl-2-yl)-3-(difluormethyl)-1-methyl-1H-pyrazol-4-carboxamid, 3-(Difluormethyl)-1-methyl-N-[4'-(trifluormethyl)biphenyl-2-yl]-1H-pyrazol-4-carboxamid, N-(2',5'-Difluorbiphenyl-2-yl)-1-methyl-3-(trifluormethyl)-1H-pyrazol-4-carboxamid, 3-(Difluormethyl)-1-methyl-N-[4'-(prop-1-in-1-yl)biphenyl-2-yl]-1H-pyrazol-4-carboxamid, 5-Fluor-1,3-dimethyl-N-[4'-(prop-1-in-1-yl)biphenyl-2-yl]-1H-pyrazol-4-carboxamid, 2-Chlor-N-[4'-(prop-1-in-1-yl)biphenyl-2-yl]pyridin-3-carboxamid, 3-(Difluormethyl)-N-[4'-(3,3-dimethylbut-1-in-1-yl)biphenyl-2-yl]-1-methyl-1H-pyrazol-4-carboxamid, N-[4'-(3,3-Dimethylbut-1-in-1-yl)biphenyl-2-yl]-5-fluor-1,3-dimethyl-1H-pyrazol-4-carboxamid, 3-(Difluormethyl)-N-(4'-ethinylbiphenyl-2-yl)-1-methyl-1H-pyrazol-4-carboxamid, N-(4'-Ethinylbiphenyl-2-yl)-5-fluor-1,3-dimethyl-1H-pyrazol-4-carboxamid, 2-Chlor-N-(4'-ethinylbiphenyl-2-yl)pyridin-3-carboxamid, 2-Chlor-N-[4'-(3,3-dimethylbut-1-in-1-yl)biphenyl-2-yl]pyridin-3-carboxamid, 4-(Difluormethyl)-2-methyl-N-[4'-(trifluormethyl)biphenyl-2-yl]-1,3-thiazol-5-carboxamid, 5-Fluor-N-[4'-(3-hydroxy-3-methylbut-1-in-1-yl)biphenyl-2-yl]-1,3-dime¬thyl-1H-pyrazol-4-carboxamid, 2-Chlor-N-[4'-(3-hydroxy-3-methylbut-1-in-1-yl)biphenyl-2-yl]pyridin-3-carboxamid, 3-(Difluormethyl)-N-[4'-(3-methoxy-3-methylbut-1-in-1-yl)biphenyl-2-yl]-1-methyl-1H-pyrazol-4-carboxamid, 5-Fluor-N-[4'-(3-methoxy-3-methylbut-1-in-1-yl)biphenyl-2-yl]-1,3-dimethyl-1H-pyrazol-4-carboxamid, 2-Chlor-N-[4'-(3-methoxy-3-methylbut-1-in-1-yl)biphenyl-2-yl]pyridin-3-carboxamid, (5-Brom-2-methoxy-4-methylpyridin-3-yl)(2,3,4-trimethoxy-6-methylphenyl)methanon, N-[2-(4-{[3-(4-Chlorphenyl)prop-2-in-1-yl]oxy}-3-methoxyphenyl)ethyl]-N2-(methylsulfonyl)valin-amid, 4-Oxo-4-[(2-phenylethyl)amino]butansäure und But-3-yn-1-yl {6-[({[(Z)-(1-methyl-1H-tetrazol-5-yl)(phenyl)methylen]amino}oxy)methyl]pyridin-2-yl}carbamat.

Alle genannten Pestizide (1) bis (16) können, wenn sie aufgrund ihrer funktionellen Gruppen dazu imstande sind, gegebenenfalls mit geeigneten Basen oder Säuren Salze bilden.

### Als Beispiele für Bakterizide seien genannt:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

Als Beispiele für Insektizide, Akarizide und Nematizide seien genannt:
(1) Acetylcholinesterase (AChE) Inhibitoren, wie beispielsweise
   Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; oder
   Organophosphate, z.B. Acephate, Azamethiphos, Azinphosethyl, Azinphosmethyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphosmethyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Antagonisten, wie beispielsweise
   Cyclodienorganochlorine, z.B. Chlordane und Endosulfan; oder
   Phenylpyrazole (Fiprole), z.B. Ethiprole und Fipronil.
(3) Natrium-Kanal-Modulatoren / Spannungsabhängige Natrium-Kanal-Blocker, wie beispielsweise
   Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)- Isomere)], Tralomethrin und Transfluthrin; oder
   DDT; oder Methoxychlor.
(4) Nikotinerge Acetylcholin-Rezeptor (nAChR) Agonisten, wie beispielsweise
   Neonikotinoide, z.B. Acetamiprid, Clothianidin, Dinotefuran, Imidacloprid, Nitenpyram, Thiacloprid und Thiamethoxam; oder
   Nikotin.
(5) Nikotinerge Acetylcholin-Rezeptor (nAChR) allosterische Aktivatoren, wie beispielsweise
   Spinosine, z.B. Spinetoram und Spinosad.
(6) Chlorid-Kanal-Aktivatoren, wie beispielsweise
   Avermectine/Milbemycine, z.B. Abamectin, Emamectin-benzoat, Lepimectin und Milbemectin.
(7) Juvenilhormon-Imitatoren, wie beispielsweise
   Juvenilhormon-Analoge, z.B. Hydroprene, Kinoprene und Methoprene; oder Fenoxycarb; oder Pyriproxyfen.
(8) Wirkstoffe mit unbekannten oder nicht spezifischen Wirkmechanismen, wie beispielsweise
   Alkylhalide, z.B. Methylbromid und andere Alkylhalide; oder
   Chloropicrin; oder Sulfurylfluorid; oder Borax; oder Brechweinstein.
(9) Selektive Fraßhemmer, z.B. Pymetrozine; oder Flonicamid.
(10) Milbenwachstumsinhibitoren, z.B. Clofentezine, Hexythiazox und Diflovidazin; oder
   Etoxazole.
(11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, Cry1Ac, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron; oder
   Organozinnverbindungen, z.B. Azocyclotin, Cyhexatin und Fenbutatin-oxid; oder Propargite; oder Tetradifon.
(13) Entkoppler der oxidativen Phoshorylierung durch Unterbrechung des H-Protongradienten, wie beispielsweise Chlorfenapyr, DNOC und Sulfluramid.
(14) Nikotinerge Acetylcholin-Rezeptor-Antagonisten, wie beispielsweise Bensultap, Cartaphydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungsstörende Wirkstoffe, Dipteran, wie beispielsweise Cyromazine.
(18) Ecdyson-Rezeptor Agonisten, wie beispielsweise Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon; oder Acequinocyl; oder Fluacrypyrim.
(21) Komplex-I-Elektronentransportinhibitoren, beispielsweise
   METI-Akarizide, z.B. Fenazaquin, Fenpyroximate, Pyrimidifen, Pyridaben, Tebufenpyrad und Tolfenpyrad; oder
   Rotenone (Derris).
(22) Spannungsabhängige Natriumkanal-Blocker, z.B. Indoxacarb; oder Metaflumizone.
(23) Inhibitoren der Acetyl-CoA-Carboxylase, wie beispielsweise Tetron- und Tetramsäurederivate, z.B. Spirodiclofen und Spiromesifen.
(24) -IV-Elektronentransportinhibitoren, wie beispielsweise
   Phosphine, z.B. Aluminiumphosphid, Calciumphosphid, Phosphin und Zinkphosphid; oder
   Cyanid.
(25) Komplex-II-Elektronentransportinhibitoren, wie beispielsweise Cyenopyrafen.
(26) Ryanodinrezeptor-Effektoren, wie beispielsweise Diamide, z.B. Chlorantraniliprole und Flubendiamide.

Weitere Wirkstoffe mit unbekanntem Wirkmechanismus, wie beispielsweise Amidoflumet, Azadirachtin, Benclothiaz, Benzoximate, Bifenazate, Bromopropylate, Chinomethionat, Cryolite, Cyantraniliprole (Cyazypyr), Cyflumetofen, Dicofol, Diflovidazin, Fluensulfone, Flufenerim, Flufiprole, Fluopyram, Fufenozide, Imidaclothiz, Iprodione, Pyridalyl, Pyrifluquinazon und lodmethan; desweiteren Präparate auf Basis von Bacillus firmus (I-1582, BioNeem, Votivo) sowie folgende bekannte wirksame Verbindungen:
3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus WO2005/077934), 4-{[(6-Brompyrid-3-yl)methyl](2-fluorethyl)amino}furan-2(5H)-on (bekannt aus WO2007/115644), 4-{[(6-Fluorpyrid-3-yl)methyl](2,2-difluorethyl)amino}furan-2(5H)-on (bekannt aus WO2007/115644), 4-{[(2-Chlor-1,3-thiazol-5-yl)methyl](2-fluorethyl)amino}furan-2(5H)-on (bekannt aus WO2007/115644), 4-{[(6-Chlorpyrid-3-yl)methyl](2-fluorethyl)amino}furan-2(5H)-on (bekannt aus WO2007/115644), 4-{[(6-Chlorpyrid-3-yl)methyl](2,2-difluorethyl)amino}furan-2(5H)-on (bekannt aus WO2007/115644), 4-{[(6-Chlor-5-fluorpyrid-3-yl)methyl](methyl)amino}furan-2(5H)-on (bekannt aus WO2007/115643), 4-{[(5,6-Dichlorpyrid-3-yl)methyl](2-fluorethyl)amino}furan-2(5H)-on (bekannt aus WO2007/115646), 4-{[(6-Chlor-5-fluorpyrid-3-yl)methyl](cyclopropyl)amino}furan-2(5H)-on (bekannt aus WO2007/115643), 4-{[(6-Chlorpyrid-3-yl)methyl](cyclopropyl)amino}furan-2(5H)-on (bekannt aus EP-A-0 539 588), 4-{[(6-Chlorpyrid-3-yl)methyl](methyl)amino}furan-2(5H)-on (bekannt aus EP-A-0 539 588), {[1-(6-Chlorpyridin-3-yl)ethyl](methyl)oxido-λ4-sulfanyliden}cyanamid (bekannt aus WO2007/149134) und seine Diastereomere {[(1R)-1-(6-Chlorpyridin-3-yl)ethyl](methyl)oxido-λ4-sulfanyliden}cyanamid (A) und {[(1S)-1-(6-Chlorpyridin-3-yl)ethyl](methyl)oxido-λ4-sulfanyliden}cyanamid (B) (ebenfalls bekannt aus WO2007/149134) sowie Sulfoxaflor (ebenfalls bekannt aus WO2007/149134) und seine Diastereomere [(R)-Methyl(oxido){(1R)-1-[6-(trifluormethyl)pyridin-3-yl]ethyl}-λ4-sulfanyliden]¬cyanamid (A1) und [(S)-Methyl(oxido){(1S)-1-[6-(trifluormethyl)pyridin-3-yl]ethyl}-λ4-sulfanyliden]cyanamid (A2), bezeichnet als Diastereomerengruppe A (bekannt aus WO 2010/074747, WO 2010/074751), [(R)-Methyl(oxido){(1S)-1-[6-(trifluormethyl)pyridin-3-yl]ethyl}-λ4-sulfanyliden]cyanamid (B1) und [(S)-Methyl(oxido){(1R)-1-[6-(trifluormethyl)pyridin-3-yl]ethyl}-λ4-sulfanyliden]cyanamid (B2), bezeichnet als Diastereomerengruppe B (ebenfalls bekannt aus WO 2010/074747, WO 2010/074751) und 11-(4-Chlor-2,6-dimethylphenyl)-12-hydroxy-1,4-dioxa-9-azadispiro[4.2.4.2]tetradec-11-en-10-on (bekannt aus WO2006/089633), 3-(4'-Fluor-2,4-dimethylbiphenyl-3-yl)-4-hydroxy-8-oxa-1-azaspiro[4.5]dec-3-en-2-on (bekannt aus WO2008/067911), 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635), [(3S,4aR,12R,12aS,12bS)-3-[(Cyclopropylcarbonyl)oxy]-6,12-dihydroxy-4,12b-dimethyl-11-oxo-9-(pyridin-3-yl)-1,3,4,4a,5,6,6a,12,12a,12b-decahydro-2H,11H-benzo[f]pyrano[4,3-b]chromen-4-yl]methylcyclopropancarboxylat (bekannt aus WO2008/066153), 2-Cyan-3-(difluormethoxy)-N,N-dimethylbenzolsulfonamid (bekannt ausWO2006/056433), 2-Cyan-3-(difluormethoxy)-N-methylbenzolsulfonamid (bekannt aus WO2006/100288), 2-Cyan-3-(difluormethoxy)-N-ethylbenzolsulfonamid (bekannt aus WO2005/035486), 4-(Difluormethoxy)-N-ethyl-N-methyl-1,2-benzothiazol-3-amin-1,1-dioxid (bekannt aus WO2007/057407), N-[1-(2,3-Dimethylphenyl)-2-(3,5-dimethylphenyl)ethyl]-4,5-dihydro-1,3-thiazol-2-amin (bekannt aus WO2008/104503), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-yl-ethylcarbonat (bekannt aus WO2009/049851), 4-(But-2-in-1-yloxy)-6-(3,5-dimethylpiperidin-1-yl)-5-fluorpyrimidin (bekannt aus WO2004/099160), (2,2,3,3,4,4,5,5-Octafluorpentyl)(3,3,3-trifluorpropyl)malononitril (bekannt aus WO2005/063094), (2,2,3,3,4,4,5,5-Octafluorpentyl)(3,3,4,4,4-pentafluorbutyl)malononitril (bekannt aus WO2005/063094), 8-[2-(Cyclopropylmethoxy)-4-(trifluormethyl)phenoxy]-3-[6-(trifluormethyl)pyridazin-3-yl]-3-azabicyclo[3.2.1]octan (bekannt aus WO2007/040280), 2-Ethyl-7-methoxy-3-methyl-6-[(2,2,3,3-tetrafluor-2,3-dihydro-1,4-benzodioxin-6-yl)oxy]chinolin-4-yl-methylcarbonat (bekannt aus JP2008/110953), 2-Ethyl-7-methoxy-3-methyl-6-[(2,2,3,3-tetrafluor-2,3-dihydro-1,4-benzodioxin-6-yl)oxy]chinolin-4-ylacetat (bekannt aus JP2008/110953), PF1364 (CAS-Reg.Nr. 1204776-60-2) (bekannt aus JP2010/018586), 5-[5-(3,5-Dichlorphenyl)-5-(trifluormethyl)-4,5-dihydro-1,2-oxazol-3-yl]-2-(1H-1,2,4-triazol-1-yl)benzonitril (bekannt aus WO2007/075459), 5-[5-(2-Chlorpyridin-4-yl)-5-(trifluormethyl)-4,5-dihydro-1,2-oxazol-3-yl]-2-(1H-1,2,4-triazol-1-yl)benzonitril (bekannt aus WO2007/075459), 4-[5-(3,5-Dichlorphenyl)-5-(trifluormethyl)-4,5-dihydro-1,2-oxazol-3-yl]-2-methyl-N-{2-oxo-2-[(2,2,2-trifluorethyl)amino]ethyl}benzamid (bekannt aus WO2005/085216), 4-{[(6-Chlorpyridin-3-yl)methyl](cyclopropyl)amino}-1,3-oxazol-2(5H)-on, 4-{[(6-Chlorpyridin-3-yl)methyl]¬(2,2-difluorethyl)amino}-1,3-oxazol-2(5H)-on, 4-{[(6-Chlorpyridin-3-yl)methyl](ethyl)amino}-1,3-oxazol-2(5H)-on, 4-{[(6-Chlorpyridin-3-yl)methyl](methyl)amino}-1,3-oxazol-2(5H)-on (alle bekannt aus WO2010/005692), NNI-0711 (bekannt aus WO2002/096882), 1-Acetyl-N-[4-(1,1,1,3,3,3-hexafluor-2-methoxypropan-2-yl)-3-isobutylphenyl]-N-isobutyryl-3,5-dimethyl-1H-pyrazol-4-carboxamid (bekannt aus WO2002/096882), Methyl-2-[2-({[3-brom-1-(3-chlorpyridin-2-yl)-1H-pyrazol-5-yl]carbonyl}amino)-5-chlor-3-methylbenzoyl]-2-methylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus WO2005/085216),Methyl-2-[3,5-dibrom-2-({[3-brom-1-(3-chlorpyridin-2-yl)-1H-pyrazol-5-yl]carbonyl}amino)benzoyl]-1,2-diethylhydrazincarboxylat (bekannt aus WO2005/085216),Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (bekannt aus WO2005/085216),(5RS,7RS;5RS,7SR)-1-(6-Chlor-3-pyridylmethyl)-1,2,3,5,6,7-hexahydro-7-methyl-8-nitro-5-propoxyimidazo[1,2-a]pyridin (bekannt aus WO2007/101369), 2-{6-[2-(5-Fluorpyridin-3-yl)-1,3-thiazol-5-yl]pyridin-2-yl}pyrimidin (bekannt aus WO2010/006713), 2-{6-[2-(Pyridin-3-yl)-1,3-thiazol-5-yl]pyridin-2-yl}pyrimidin (bekannt aus WO2010/006713), 1-(3-Chlorpyridin-2-yl)-N-[4-cyan-2-methyl-6-(methylcarbamoyl)phenyl]-3-{[5-(trifluormethyl)-1H-tetrazol-1-yl]methyl}-1H-pyrazol-5-carboxamid (bekannt aus WO2010/069502), 1-(3-Chlorpyridin-2-yl)-N-[4-cyan-2-methyl-6-(methylcarbamoyl)phenyl]-3-{[5-(trifluormethyl)-2H-tetrazol-2-yl]methyl}-1H-pyrazol-5-carboxamid (bekannt aus WO2010/069502), N-[2-(tert-Butylcarbamoyl)-4-cyan-6-methylphenyl]-1-(3-chlorpyridin-2-yl)-3-{[5-(trifluormethyl)-1H-tetrazol-1-yl]methyl}-1H-pyrazol-5-carboxamid (bekannt aus WO2010/069502), N-[2-(tert-Butylcarbamoyl)-4-cyan-6-methylphenyl]-1-(3-chlorpyridin-2-yl)-3-{[5-(trifluormethyl)-2H-tetrazol-2-yl]methyl}-1H-pyrazol-5-carboxamid (bekannt aus WO2010/069502) und (1E)-N-[(6-Chlorpyridin-3-yl)methyl]-N'-cyan-N-(2,2-difluorethyl)ethanimidamid (bekannt aus WO2008/009360).

Die hier mit ihrem "common name" genannten Wirkstoffe sind bekannt und beispielsweise im Pestizid handbuch ("The Pesticide Manual" 16th Ed., British Crop Protection Council 2012) beschrieben oder im Internet recherchierbar (z.B. http://www.alanwood.net/pesticides).

Bei den Pestiziden der Komponente c) kann es sich auch um eine Kombination von zwei oder mehr Pestiziden handeln. Solche Kombinationen sind insbesondere dann von Bedeutung, wenn es beispielsweise darum geht, das Wirkungsspektrum der Pestizid-Zusammensetzung zu verbreitern oder Resistenzen gegenüber bestimmte Pestizide besser zu unterbinden.

In einer bevorzugten Ausführungsform der Erfindung beträgt die Menge des einen oder der mehreren Pestizide der Komponente c) in den erfindungsgemäßen Zusammensetzungen mehr als 100 g/l, bevorzugt mehr als 200 g/l und besonders bevorzugt mehr als 300 g/l. Diese Mengenangaben beziehen sich auf das Gesamtgewicht der erfindungsgemäßen Pestizid-Zusammensetzung und im Falle von Pestiziden, die in Form ihrer wasserlöslichen Salze eingesetzt werden auf die Menge an freier Säure, dem sogenannten Säureäquivalent ("acid equivalent", a.e.).

Der pH-Wert der Pestizidzusammensetzungen liegt üblicherweise im Bereich von 3,5 bis 8,5, bevorzugt bei 4,0 bis 8,0 und besonders bevorzugt bei 4,5 bis 6,5 (gemessen als 1 gew.-%ige wässrige Verdünnung). Der pH-Wert wird primär bestimmt durch die pH-Werte der Lösungen der wässrigen Pestizide, die als Salze schwacher Säuren vorliegen. Durch Zugabe von Säuren oder Basen kann der pH-Wert auf einen anderen Wert abweichend von dem ursprünglichen pH-Wert der Mischung eingestellt werden.

In einer weiteren bevorzugten Ausführungsform der Erfindung liegen die erfindungsgemäßen Pestizidzusammensetzungen als Konzentrat-Formulierungen vor, die vor dem Gebrauch verdünnt werden, insbesondere mit Wasser (beispielsweise "ready-to-use"-, "in-can"- oder "built-in"-Formulierungen), und enthalten das eine oder die mehreren Ammoniumsalze (I) im Allgemeinen in Mengen von 5 bis 80 Gew.-%, bevorzugt von 10 bis 70 Gew.-% und besonders bevorzugt von 20 bis 60 Gew.-%. Diese Mengenangaben beziehen sich auf die gesamte Konzentrat-Formulierung.

Die erfindungsgemäßen Pestizidzusammensetzungen werden vorzugsweise in Form von Spritzbrühen auf die Felder ausgebracht. Dabei werden die Spritzbrühen durch Verdünnung von Konzentrat-Formulierungen mit einer definierten Menge Wasser hergestellt.

In einer weiteren bevorzugten Ausführungsform der Erfindung liegen die erfindungsgemäßen Pestizidzusammensetzungen als Spritzbrühen vor und enthalten 0,001 bis 10 Gew.-%, bevorzugt 0,02 bis 3 Gew.-% und besonders bevorzugt 0,025 bis 2 Gew.-% des einen oder der mehreren Ammoniumsalze (I) Komponente a).

Die Erfindung betrifft weiterhin die Verwendung der erfindungsgemäßen Pestizidzusammensetzungen zur Kontrolle und/oder zur Bekämpfung von Unkraut, Pilzerkrankungen oder Insektenbefall. Bevorzugt ist die Verwendung der erfindungsgemäßen Zusammensetzungen zur Kontrolle und/oder zur Bekämpfung von Unkraut.

Diese Verwendungen können vorzugsweise auch im sogenannten Tank-mix-Verfahren stattfinden. Hierbei können also das oder die mehreren wasserlöslichen Pestizide der Komponente g) und die Komponenten a) bis d) sowie zusätzlich Wasser auch in Form einer sogenannten "Tank-mix"- Zubereitung vorliegen. In einer derartigen Zubereitung liegen sowohl das oder die mehreren wasserlöslichen Pestizide als auch die Komponenten a) bis d), letztere gegebenenfalls zusammen mit weiteren Adjuvants, getrennt voneinander vor. Beide Zubereitungen werden vor dem Ausbringen, in der Regel kurz vorher, miteinander vermischt, wobei eine erfindungsgemäße Pestizid-Zusammensetzung entsteht.

Gegenstand der Erfindung ist auch ein Verfahren zur Bekämpfung von Schadorganismen, wobei man den Schadorganismus oder dessen Lebensraum mit einem erfindungsgemäßen Ammoniumsalz (I) oder einer erfindungsgemäßen Pestizidzusammensetzung in Kontakt bringt. Bei den Schadorganismen handelt es sich vorzugsweise um unerwünschte Pflanzen.

### Ausführungsbeispiele

### Herstellung

### Herstellungsbeispiel 1 (nicht erfindungsgemäss)

37 g Dicamba wurden unter Rühren in 30 g deionisiertem Wasser suspendiert. Anschließend wurden unter Rühren 33 g N-Methylglucamin in die Wirkstoffsuspension eingetragen. Es entstand eine klare Lösung.

### Herstellungsbeispiel 2 (nicht erfindungsgemäss)

37 g Dicamba wurden unter Rühren in 30 g deionisiertem Wasser suspendiert. Anschließend wurden unter Rühren 33 g Dimethylglucamin in die Wirkstoffsuspension eingetragen. Es entstand eine klare Lösung.

### Herstellungsbeispiel 3 (nicht erfindungsgemäss)

21 g MCPA wurden unter Rühren in 58 g deionisiertem Wasser suspendiert. Anschließend wurden unter Rühren 21 g N-Methylglucamin in die Wirkstoffsuspension eingetragen. Es entstand eine klare Lösung.

### Herstellungsbeispiel 4

20 g Pelargonsäure wurden unter Rühren in 55 g deionisiertem Wasser suspendiert. Anschließend wurden unter Rühren 25 g N-Methylglucamin in die Wirkstoffsuspension eingetragen. Es entstand eine klare Lösung.

### Herstellungsbeispiel 5

20 g Pelargonsäure wurden unter Rühren in 10 g deionisiertem Wasser suspendiert. Anschließend wurden unter Rühren 25 g Dimethylglucamin in die Wirkstoffsuspension eingetragen. Es entstand eine klare Lösung.

### Herstellungsbeispiel 6 (nicht erfindungsgemäss)

0.21 g Mesotrione wurden unter Rühren in 10 g deionisiertem Wasser suspendiert. Anschließend wurden unter Rühren 0.11 g N-Methylglucamin in die Wirkstoffsuspension eingetragen. Es entstand eine klare Lösung.

### Herstellungsbeispiel 7 (nicht erfindungsgemäss)

0.13 g Nicosulfuron wirden unter Rühren in 10 g deionisiertem Wasser suspendiert. Anschließend wurden unter Rühren 0.07 g N-Methylglucamin in die Wirkstoffsuspension eingetragen. Es entstand eine klare Lösung.

### Herstellungsbeispiel 8 (nicht erfindungsgemäss)

0.11 g Nicosulfuron wurden unter Rühren in 10 g deionisiertem Wasser suspendiert. Anschließend wurden unter Rühren 0.065 g Dimethylglucamin in die Wirkstoffsuspension eingetragen. Es entstand eine klare Lösung.

Zu beachten ist, dass nur die Herstellungsbeispiele 4 und 5 von den Ansprüchen umfasst sind, die Herstellungsbeispiele 1, 2, 3, 6, 7 und 8 sind kein Bestandteil der vorliegenden Erfindung.

### Formulierungen

Die Zusammensetzung der Wirkstoffformulierungen aus den Herstellungsbeispielen sind in Tabelle 1 zusammengefasst:

**Tabelle 1**

| Zusammensetzung | Beispiel (w%] | | | | | | | |
|---|---|---|---|---|---|---|---|---|
| | 1 | 2 | 3 | 4 | 5 | 6 | 7 | 8 |
| Dicamba | 37 | 37 | 0 | 0 | 0 | 0 | 0 | 0 |
| MCPA | 0 | 0 | 21 | 0 | 0 | 0 | 0 | 0 |
| Pelargonsäure | 0 | 0 | 0 | 20 | 20 | 0 | 0 | 0 |
| Mesotrione | 0 | 0 | 0 | 0 | 0 | 2 | 0 | 0 |
| Nicosulfuron | 0 | 0 | 0 | 0 | 0 | 0 | 1.3 | 1.1 |
| NMG | 33 | 0 | 21 | 25 | 0 | 1 | 0.7 | 0 |
| DMG | 0 | 33 | 0 | 0 | 25 | 0 | 0 | 0.6 |
| Wasser | 30 | 30 | 58 | 55 | 55 | 97 | 98 | 98.3 |
| Aussehen | Klare Lösung | Klare Lösung | Klare Lösung | Klare Lösung | Klare Lösung | Klare Lösung | Klare Lösung | Klare Lösung |

| | | | | | | | | |
|---|---|---|---|---|---|---|---|---|
| NMG = N-Methylglucamin DMG = Dimethylglucamin | | | | | | | | |

## Patentansprüche

1. Organisches Ammoniumsalz der Formel (I), 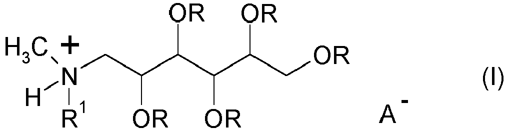 wobei die Symbole folgende Bedeutungen haben:
A⁻ ist ein anionisches Pestizid,
R¹ ist C₁-C₄-Alkyl, CH₂CH₂OH oder CH₂CH(CH₃)OH;
R ist H, CH₃ oder zwei benachbarte Reste R bilden zusammen eine Gruppe -C(R')₂- und
R' ist gleich oder verschieden H oder CH₃,
**dadurch gekennzeichnet, dass** A ausgewählt ist aus Octansäure, Pelargonsäure und Ölsäure.

2. Ammoniumsalz gemäß Anspruch 1, **dadurch gekennzeichnet, dass** A Pelargonsäure ist.

3. Ammoniumsalz gemäß Anspruch 1 oder 2, wobei die Symbole in der Formel (I) folgende Bedeutungen haben:
R¹ ist C₁-C₄-Alkyl oder CH₂CH₂OH und
R ist H oder CH₃.

4. Ammoniumsalz gemäß einem der Ansprüche 1 bis 3, wobei R¹ = CH₃ und R = H ist oder R¹ = CH₂CH₂OH und R = H ist.

5. Ammoniumsalz gemäß einem der Ansprüche 1 bis 4, wobei R¹ = CH₃ ist und R = H ist.

6. Ammoniumsalz gemäß einem der Ansprüche 1 bis 5, wobei A = Pelargonsäure ist, R¹ = CH₃ ist und R = H ist.

7. Verfahren zur Herstellung eines Ammoniumsalzes gemäß einem der Ansprüche 1 bis 6, wobei man die protonierte Form des anionischen Pestizids A⁻ mit einem Glucamin der Formel (II) umsetzt, 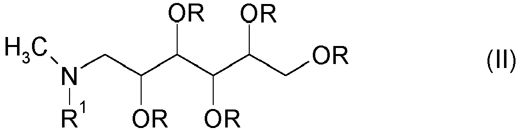 wobei die Symbole die in der Formel (I) angegebenen Bedeutungen haben.

8. Nicht-therapeutische Verwendung eines Ammoniumsalzes gemäß einem der Ansprüche 1 bis 6 als Pestizid.

9. Pestizidzusammensetzung, enthaltend
a) ein oder mehrere Ammoniumsalze (I) gemäß einem der Ansprüche 1 bis 6 und
b) einen oder mehrere Formulierungshilfsstoffe.

10. Pestizidzusammensetzung gemäß Anspruch 9 in Form einer Herbizidzusammensetzung.

11. Pestizidzusammensetzung gemäß Anspruch 9 oder 10, enthaltend als Formulierungshilfsstoff b) ein oder mehrere Copolymere A), wobei die Copolymere eine oder mehrere Struktureinheiten, hervorgegangen aus
a) 19,9 bis 75,9 Gew.-% Glycerin
b) 0,1 bis 30 Gew.-% mindestens einer Dicarbonsäure und
c) 24 bis 80 Gew.-% mindestens einer Monocarbonsäure gemäß Formel (III),
R²-COOH (III)
wobei R² (C₅-C₂₉)-Alkyl; (C₇-C₂₉)-Alkenyl; Phenyl oder Naphthyl darstellt, enthalten.

12. Nicht-therapeutisches Verfahren zur Bekämpfung von Schadorganismen, wobei man den Schadorganismus oder dessen Lebensraum mit einem Ammoniumsalz (I) gemäß einem der Ansprüche 1 bis 6 oder einer Pestizidzusammensetzung gemäß einem der Ansprüche 9 bis 11 in Kontakt bringt.

13. Verfahren gemäß Anspruch 12, wobei es sich bei den Schadorganismen um unerwünschte Pflanzen handelt.

14. Verwendung eines Ammoniumsalzes gemäß einem der Ansprüche 1 bis 6 zur Verminderung der Flüchtigkeit des als Salz vorliegenden anionischen Pestizids.

## Claims

1. Organic ammonium salt of the formula (I) 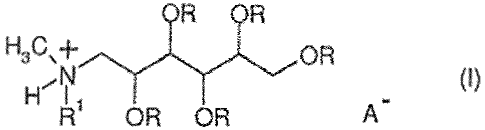 where the symbols have the following definitions:
A⁻ is an anionic pesticide,
R¹ is C₁-C₄-alkyl, CH₂CH₂OH or CH₂CH(CH₃)OH;
R is H, CH₃, or two adjacent R radicals together form a -C(R')₂- group and
R' is the same or different and is H or CH₃,
**characterized in that** A is selected from octanoic acid, pelargonic acid and oleic acid.

2. Ammonium salt according to Claim 1, **characterized in that** A is pelargonic acid.

3. Ammonium salt according to Claim 1 or 2, where the symbols in the formula (I) have the following definitions:
R¹ is C₁-C₄-alkyl or CH₂CH₂OH and
R is H or CH₃.

4. Ammonium salt according to any of Claims 1 to 3, where R¹ = CH₃ and R = H or R¹ = CH₂CH₂OH and R = H.

5. Ammonium salt according to any of Claims 1 to 4, where R¹ = CH₃ and R = H.

6. Ammonium salt according to any of Claims 1 to 5, where A = pelargonic acid, R¹ = CH₃ and R = H.

7. Process for preparing an ammonium salt according to any of Claims 1 to 6, wherein the protonated form of the anionic pesticide A⁻ is reacted with a glucamine of the formula (II) 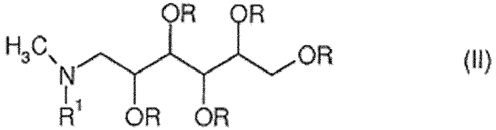 where the symbols have the definitions given in the formula (I).

8. Non-therapeutic use of an ammonium salt according to any of Claims 1 to 6 as a pesticide.

9. Pesticide composition comprising
a) one or more ammonium salts (I) according to any of Claims 1 to 6
and
b) one or more formulation auxiliaries.

10. Pesticide composition according to Claim 9 in the form of a herbicide composition.

11. Pesticide composition according to Claim 9 or 10, comprising, as formulation auxiliary b), one or more copolymers A), where the copolymers contain one or more structural units derived from
a) 19.9% to 75.9% by weight of glycerol
b) 0.1% to 30% by weight of at least one dicarboxylic acid and
c) 24% to 80% by weight of at least one monocarboxylic acid of formula (III)
R²-COOH (III)
where R² is (C₅-C₂₉)-alkyl; (C₇-C₂₉)-alkenyl; phenyl or naphthyl.

12. Non-therapeutic method of controlling harmful organisms, wherein the harmful organism or its habitat is brought into contact with an ammonium salt (I) according to any of Claims 1 to 6 or a pesticide composition according to any of Claims 9 to 11.

13. Method according to Claim 12, where the harmful organisms are unwanted plants.

14. Use of an ammonium salt according to any of Claims 1 to 6 for reducing the volatility of the anionic pesticide present in salt form.

## Revendications

1. Sel d'ammonium organique de formule (I) 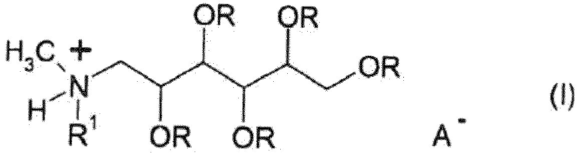
dans laquelle les symboles ont les significations suivantes :
A⁻ est un pesticide anionique,
R¹ représente alkyle en C₁-C₄, CH₂CH₂OH ou CH₂CH(CH₃)OH ;
R représente H, CH₃, ou deux radicaux R voisins forment ensemble un groupe -C(R')₂- et
les R' représentent, de manière identique ou différente, H ou CH₃,
**caractérisé en ce qu'**A est choisi parmi l'acide octanoïque, l'acide pélargonique et l'acide oléique.

2. Sel d'ammonium selon la revendication 1, **caractérisé en ce qu'**A est l'acide pélargonique.

3. Sel d'ammonium selon la revendication 1 ou 2, **caractérisé en ce que** les symboles dans la formule (I) ont les significations suivantes :
R¹ représente alkyle en C₁-C₄ ou CH₂CH₂OH et
R représente H ou CH₃.

4. Sel d'ammonium selon l'une quelconque des revendications 1 à 3, dans lequel R¹ = CH₃ et R = H ou R¹ = CH₂CH₂OH et R = H.

5. Sel d'ammonium selon l'une quelconque des revendications 1 à 4, dans lequel R¹ = CH₃ et R = H.

6. Sel d'ammonium selon l'une quelconque des revendications 1 à 5, dans lequel A = acide pélargonique, R¹ = CH₃ et R = H.

7. Procédé de fabrication d'un sel d'ammonium selon l'une quelconque des revendications 1 à 6, dans lequel la forme protonée d'un pesticide anionique A⁻ est mise en réaction avec une glucamine de formule (II) 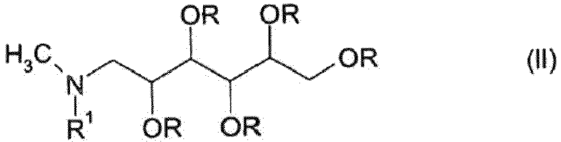 dans laquelle les symboles ont les significations indiquées dans la formule (I).

8. Utilisation non thérapeutique d'un sel d'ammonium selon l'une quelconque des revendications 1 à 6 en tant que pesticide.

9. Composition pesticide, contenant :
a) un ou plusieurs sels d'ammonium (I) selon l'une quelconque des revendications 1 à 6, et
b) un ou plusieurs adjuvants de formulation.

10. Composition pesticide selon la revendication 9, sous la forme d'une composition herbicide.

11. Composition pesticide selon la revendication 9 ou 10, contenant en tant qu'adjuvants de formulation b) un ou plusieurs copolymères A), les copolymères contenant une ou plusieurs unités structurales, dérivées de :
a) 19,9 à 75,9 % en poids de glycérine,
b) 0,1 à 30 % en poids d'au moins un acide dicarboxylique et
c) 24 à 80 % en poids d'au moins un acide monocarboxylique selon la formule (III)
R²-COOH (III)
dans laquelle R² représente alkyle en (C₅-C₂₉) ; alcényle en (C₇-C₂₉) ; phényle ou naphtyle.

12. Procédé non thérapeutique de lutte contre des organismes nuisibles, dans lequel l'organisme nuisible ou son habitat est mis en contact avec un sel d'ammonium (I) selon l'une quelconque des revendications 1 à 6 ou une composition pesticide selon l'une quelconque des revendications 9 à 11.

13. Procédé selon la revendication 12, dans lequel les organismes nuisibles sont des plantes indésirables.

14. Utilisation d'un sel d'ammonium selon l'une quelconque des revendications 1 à 6 pour réduire la volatilité du pesticide anionique présent sous la forme d'un sel.
